# EUROPEAN PATENT APPLICATION

(11) **EP 2 740 500 A2**
(43) Date of publication of application: **11.06.2014**
(21) Application number: 13195112.1
(22) Date of filing: 29.11.2013
(51) Int. Cl.: A61L 31/00, A61L 31/14

(54) **Composition for use in the prophylaxis of post-surgical adhesions**

(30) Priority: 05.12.2012 DE 102012222365
(71) Applicant: AESCULAP AG, 78532 Tuttlingen (DE)
(72) Inventor: Odermatt, Erich, 8200 Schaffhausen (CH); Bargon, Rainer, 78532 Tuttlingen (DE)
(74) Representative: Patentanwälte Ruff, Wilhelm, Beier, Dauster & Partner

(57) **Abstract**

The invention relates to a composition, preferably in the form of a hydrogel, for use in the prevention and/or prophylaxis of post-surgical tissue adhesions, comprising a moisturizing component, a mucoadhesive component, a component obtained from *Aloe vera* and water.

The invention further relates to a kit for use in the prevention and/or prophylaxis of post-surgical tissue adhesions, comprising, in addition to the composition, at least one further component which is selected from the group consisting of a medical adhesive, a disinfectant, an antibiotic, a cytostatic, an agent promoting wound healing, an implant for tissue regeneration, a haemostyptic, a one-, two-, or three-dimensional textile implant such as, for example, a suture material, a hernia mesh or a plug, an application aid, an open applicator, an applicator for laparoscopic purposes, and combinations thereof.

## Description

### Field of Application and Prior Art

The invention relates to a composition and also a kit for use in the prophylaxis of post-surgical adhesions.

Tissue adhesions are taken to mean generally the formation of fibrin strands between opposing damaged tissue structures. Adhesions can occur, for example, in the abdominal cavity, in the pelvic cavity, in particular in the region of the internal female genital organs, on the pericardium, and also on wounded or damaged nerves.

Manifestation of unwanted tissue growths or tissue adhesions after surgical interventions is already a long-known problem in surgical medical care (Treutner K-H, Schumpelick V (2000) Adhäsionsprophylaxe: Wünsche und Wirklichkeit. [Adhesion prophylaxis: wishes and reality.] Chirurg 2000; 71: 510-517).

Post-surgical tissue adhesions can have serious consequences for the patients affected. For example, mechanical ileus (bowel obstruction), a frequently occurring consequence of post-surgical adhesions, is fatal in 10% of cases (Menzies D, Parker M, Hoare R, Knight A (2001) Small bowel obstruction due to postoperative adhesions: treatment patterns and associated costs in 110 hospital admissions. Ann. R. Coll. Surg. Engl. 83: 40-46). In addition, the occurrence of post-surgical adhesions in about 20% of women infertile after an operation is considered to be causative for the infertility (Gomel V (2005) Reproductive surgery. Minerva Ginecol 57(1): 21-28). In addition, growths between the epicardium and pericardium, in particular in children, frequently have a fatal course, since the resternotomy required in these cases involves a high risk that the large cardiac vessels of the affected patients become damaged (Lodge AJ et al. (2008) A Novel Bioresorbable Film Reduces Postoperative Adhesions After Infant Cardiac Surgery. Ann. Thorac. Surg. 86: 614-621).

A survey, in particular with respect to the formation and pathology of post-surgical adhesions, is given by Brüggmann *et al.* (Brüggmann D, Tchartchian G, Wallwiener M, Münstedt K, Tinneberg H-R, Hackethal A: (2010) Intraabdominal adhesions. Dtsch Ärztebl Int. 107(44): 769-775). Against this background, in the meantime, various treatment approaches have been developed which are said to prevent or at least reduce the occurrence of post-surgical adhesions.

A pharmacological treatment approach consists in administering non-steroidal, anti-inflammatory drugs, or corticosteroids, in order to intervene directly in the enzymatic coagulation cascade. Disadvantages in principle are the dependence of the success of treatment on the administered dose, the high risk of bleeding and also the occurrence of wound-healing disorders.

A further treatment approach is the application of liquids (instillation) in order to prevent the formation of tissue adhesions by hydroflotation.

A further treatment approach relates to the use of membranes and gels which act as mechanical adhesion barriers and keep tissue structures that are at risk of adhesion apart from one another at least temporarily. General functioning principles of adhesion prophylaxis are subject matter of the survey article by Trew G (Trew G (2006) Adhesion reduction options. Future Directions In Surgery: 31-34).

Polyvinyl alcohol hydrogels (PVA hydrogels) having anti-adhesive properties for use in human or veterinary medicine are known, for example, from DE 10 2009 029 717 A1.

An approach known from the documents WO 1997/022371 A1 and US 8,067,028 B2 for producing mechanical adhesion barriers is based on the polymerization of an activated ester with a nucleophilic reaction partner, in particular a polyamine or a polyalcohol. Further concepts, for example known from the publications EP 1 292 316 B1 and US 6,696,499 B1, are based on the use of polyalkylene oxides. The use of hyaluronic acid formulations or polyurethanes as adhesion barriers is known from WO 2005/089472 A2 and US 8,071,663 B2. The adhesion barriers described there can be used as film, solution, hydrogel, sealing means or nonwoven web.

GB 2488915 A relates to a haemostatic gel made of a crosslinked acrylic acid polymer and a chitosan salt which is applied to a compression bandage for wound treatment.

The document CA 1,295,242 discloses a pharmaceutical formulation for topical use, which, in addition to α-interferon, comprises a surfactant as anti-adhesive agent and also polyacrylic acid as a carrier substance.

A self-adhesive hydratable polymer matrix in the form of a flat wound dressing or a film is described by EP 1 328 300 B1 which can comprise polyacrylic acid as structural polymer, a cellulose derivative or polyvinylpyrrolidone as adhesive component and glycerol as plasticizer.

A frequently occurring problem in particularly gel-type antiadhesion products is that they do not permit, or permit only inadequate, tissue adhesion, and can therefore move from an operation site. A further problem is non-transparent gels, which make assessment of the operation site after their application difficult, or wholly impossible. A further problem relates to the heat or cold lability. Transport and storage difficulties are involved herewith and the product stability is endangered. In some gels, this temperature sensitivity is accompanied by viscosity variations. For instance, in particular decreases in viscosity can lead to the gels or precursor components thereof being only deliverable under conditions which are more difficult, or else the gels, after delivery thereof, having a tendency to dislocation.

Some gels also suffer from the disadvantage that they exhibit excessively fast bioresorption. In addition, acidic degradation products, in particular in the case of gels based on polyester copolymers, may cause tissue irritation, and poorly degradable gels may lead to excess granulation tissue and/or a tissue encapsulation of the poorly resorbable material with known consequences. Both can permanently impair the efficacy of the gels.

Further disadvantages can occur from the fact that gel components, to avoid premature gelation, frequently need to be stored spatially separated from one another and be first mixed with one another *in situ,* obtaining a gel. Gelation occurring with a time delay can, in these cases, then be causative for unwanted dislocations and also for the occurrence of inhomogeneities in the gelled product as a result of local concentration differences, or else the aid used for application (for example, syringe or applicator) can block and prevent uniform application of the hydrogel.

### Object and Solution

Against this background, the object of the invention, therefore, was to provide a composition for the avoidance or prophylaxis of unwanted tissue adhesions or tissue adherences, which avoids shortcomings known from the prior art.

This object is achieved according to the invention by a composition having the features of independent Claim 1. Preferred embodiments of the composition are subject matter of dependent Claims 2 to 12. A further aspect of the invention relates to a kit having the features of Claim 13. The wording of all the claims is hereby made content of the present description by explicit reference.

The invention relates to a composition, preferably in the form of a hydrogel, for use in the prevention and/or prophylaxis of post-surgical or post-operative tissue adhesions or tissue adherences.

The composition is distinguished particularly in that it comprises a moisturizing component, a mucoadhesive component, a component obtained from *Aloe vera* and water.

The invention is based on the surprising finding that a composition having a moisturizing component, a mucoadhesive component, a component obtained from *Aloe vera* and water as antiadhesion agent or adhesion barrier can be used for the prevention and/or prophylaxis of post-surgical or post-operative tissue adhesions or tissue adherences.

The expression "moisturizing component", for the purposes of the present invention, is to be taken to mean a compound which is hygroscopic per se, or a mixture of hygroscopic compounds, which, however, owing to the hygroscopic properties thereof, contains or contain bound water (as hydration shell), in particular via the development of hydrogen bonds, and can release this bound water to tissue.

In a preferred embodiment, the moisturizing component is a compound bearing hydroxyl groups.

Particularly preferably, the moisturizing component is a polyol, in particular triol or diol.

For example, the moisturizing component can be selected from the group consisting of ethylene glycol, propylene glycol, butylene glycol, glycerol, sorbitol, xylitol and combinations thereof.

Glycerol is particularly preferred as moisturizing component.

Preferably, the composition has a moisturizing component fraction of at least 5% by weight, in particular 5% by weight to 20% by weight, preferably 5% by weight to 15% by weight, particularly preferably 8% by weight to 12% by weight, based on the total weight of the composition.

The expression "mucoadhesive component", for the purposes of the present invention, is to be taken to mean a compound or a mixture of compounds which is or are able to bind water in the form of hydration shells and to enter into an adhesive bond to the mucin layer of mucosae. The mucin layer consists fundamentally of glycoproteins (mucins) which have sulphate and carboxylic acid groups and as a result thereof can bind large amounts of water in the form of hydration shells. The binding of the mucoadhesive component to a mucin layer therefore arises in that water is "trapped" firstly by mucins and secondly by the mucoadhesive component, and a molecular bond is formed between the mucin layer and the mucoadhesive component, in particular via electrostatic interactions and dipole-dipole interactions. Therefore, the mucoadhesive component contributes to the composition being fixed in the position of an operation site or wound site sufficiently long to prevent the occurrence of tissue adhesions.

The mucoadhesive component is preferably a compound bearing carboxyl groups, more preferably a polycarboxylic acid, in particular polyacrylic acid or a polysaccharide bearing carboxyl groups, such as, for example, carboxymethylcellulose (CMC). Particularly preferably it is poly-acrylic acid. An advantage of polyacrylic acid is, in particular, that it can act as a flow limiter and can increase in this manner the structural viscosity or viscosity at rest of the composition (viscosity of the composition in the resting state at room temperature). A suitable polyacrylic acid is commercially available, for example, under the name Carbopol 940.

In a further embodiment, the mucoadhesive component is a polycarboxylic acid, preferably polyacrylic acid, having a mean molecular weight from 1 kDa to 10 000 kDa (kilo Dalton), in particular from 1 kDa to 1000 kDa, or 10 kDa to 2000 kDa, preferably from 50 kDa to 150 kDa.

In a further embodiment, the mucoadhesive component is a polyacrylic acid of (about) 1450 acrylic acid monomer units.

In a further embodiment, the composition has a mucoadhesive component content of at least 0.1% by weight, in particular from 0.1% by weight to 5% by weight, preferably 0.5% by weight to 3% by weight, particularly preferably 1% by weight to 2% by weight, based on the total weight of the composition.

In a further embodiment, the component obtained from *Aloe vera* is at least one constituent characteristic of the plant, in particular a mixture of such constituents.

In particular, the component obtained from *Aloe vera* can be a constituent acting as binder, or a mixture of such constituents.

The component obtained from *Aloe vera* can be, for example, a constituent of *Aloe vera* which is selected from the group consisting of monosaccharides, homopolysaccharides, heteropolysaccharides, anthracene derivatives and/or anthraquinone derivatives, anthrone derivatives, chromone derivatives, pyrones (pyranones), coumarins, alkaloids, glycoproteins, vitamins, vitamin precursor compounds, amino acids, peptides, polypeptides, enzymes such as, for example, amylase, alkaline phosphatase and lipase, salicylic acid, phytohormones and combinations thereof.

Suitable monosaccharides can be selected, for example, from the group consisting of arabinose, galactose, glucose, mannose, xylose and combinations thereof.

Suitable heteropolysaccharides which may be mentioned are, for example, glucomannans and/or acemannans.

Suitable anthracene derivatives and/or anthraquinone derivatives can be selected from the group comprising aloin A, aloin B (barbaloin A and B), emodin, aloe-emodin and also glycosidic derivatives thereof (termed glyco-aloe-emodin-anthrones), nataloin, homonataloin, isobarbaloin, aloesin and the aloinosides A and B derived therefrom by glycosidic links, chrysophanol and combinations thereof.

With respect to further suitable constituents which can be obtained from *Aloe vera* and can come into consideration according to the invention for the component obtained from *Aloe vera,* reference is made to the article by E. Dagne *et al.* (Dagne E., Bisrat D., Viljoen A., Van Wyk B-E. (2000) Chemistry of Aloe Species. Curr. Org. Chem. 4: 1055-1078), the disclosure of which with respect to the constituents cited therein is made contents of the present description by explicit reference.

In a further embodiment, the component obtained from *Aloe vera* is selected from the group consisting of *Aloe vera* gel, *Aloe vera* extract, *Aloe vera* essence, *Aloe vera* powder and combinations thereof.

The variants cited in the preceding paragraph for the component obtained from *Aloe vera* contain at least one, but preferably a mixture, of the constituents of *Aloe vera* described in the preceding embodiments.

The component obtained from *Aloe vera,* in a particularly preferred embodiment, is present as a gel, that is to say as what is termed *Aloe vera* gel. A suitable gel is commercially available, for example, under the name *Aloe vera* gel 5X.

The expression *"Aloe vera* gel", for the purposes of the present invention, shall be taken to mean a gel obtained from the water storage tissue of leaves of *Aloe vera.* Owing to a content of polysaccharides principally composed of D-glucose and D-mannose, the gel has a mucous consistency. In addition, the gel can contain other constituents, as described, for example, in the preceding embodiments, in particular simple sugars such as glucose, mannose, galactose and xylose, and also water-soluble vitamins, amino acids, glycoproteins, amylase, alkaline phosphatase, lipase, salicylic acid and optionally aloenins.

The *"Aloe vera* gel" can be, in particular, the viscous or highly viscous gel-type and liquid leaf gel, preferably the fresh leaf gel, which is obtainable, for example, by pressing and/or stirring. The leaves of *Aloe vera* used for producing the gel can in addition be hand peeled and, for example, freed from the leaf sheath which is aloin-containing. The gel can in addition have a water fraction of > 90% by weight, in particular a water fraction from 92% by weight to 98% by weight, based on the total weight of the gel.

To obtain the *Aloe vera* gel, methods differing in principle come into consideration, for example the whole leaf method, the roller method, the mechanical peeling method, and also filleting of the leaves by hand.

The three first mentioned methods are industrial methods in which the crude product, depending on the method, can still have relatively large fractions of *Aloe vera* juice. Filtration steps can be provided for removing the aloin present in the *Aloe vera* juice and having a relaxant activity. Mechanical methods for producing *Aloe vera* gel are known, for example, from documents US 4,488,482 and US 4,555,987.

The manual filleting of *Aloe vera* leaves is a particularly gentle method for obtaining the leaf marrow or an *Aloe vera* gel originating from the leaf marrow.

The type of production of a component obtained from *Aloe vera* in the form of an *Aloe vera* gel is not critical in the context of the present invention. Either mechanical or manual methods can be used. However, under some circumstances, it can be desirable to use a component obtained from *Aloe vera* in the form of an *Aloe vera* gel which is free from constituents having a relaxant activity such as aloin A and/or aloin B. "Substantially free" in this case shall be taken to mean a concentration of less than 0.0001% by weight of aloin A and/or aloin B, based on the total weight of the gel.

The expression *"Aloe vera* extract", for the purposes of the present invention, shall be taken to mean an extract of *Aloe vera,* that is to say the result of a workup concentrating the constituents of the leaves and/or other components of the plant and, in particular, reducing the volume. The *Aloe vera* extract can, alternatively, also be obtained from an *Aloe vera* gel.

Preferred extraction media which may be mentioned are water, alcohols, terpenes, diethyl ether, vegetable oils, and also chlorinated hydrocarbons or n-hexane. Water and alcohols are particularly preferred. The constituents characteristic of *Aloe vera* are generally present unchanged in the extraction medium after the extraction process. The production of the *Aloe vera* extract can proceed, for example, according to the extraction method disclosed in EP 1 952 817 A1.

Optionally, it can be preferred to concentrate the *Aloe vera* extract further after it is obtained. This can be achieved in the simplest case by slow evaporation of the extraction medium. In this context, then, this is also termed an *Aloe vera* concentrate.

The *Aloe vera* powder can be, for example, a dry extract or a dry concentrate which - as already mentioned above - can be obtained, for example, by complete removal, optionally in vacuum, of an extraction medium. Alternatively, the *Aloe vera* powder can be dried, in particular freeze-dried, leaf and/or plant parts, which are present comminuted in powder form.

The component obtained from *Aloe vera,* in a further embodiment, has a fraction of at least 0.1% by weight, in particular from 0.1% by weight to 5% by weight, preferably 0.5% by weight to 3% by weight, particularly preferably 1% by weight to 2% by weight, based on the total weight of the composition.

The water provided in the composition according to the invention can have a fraction of at least 65% by weight, in particular from 65% by weight to 90% by weight, preferably 80% by weight to 90% by weight, particularly preferably 80% by weight to 85% by weight, based on the total weight of the composition.

In a further embodiment, the composition has an additional component selected from the group consisting of chelating agents, preservatives, detergent builders, active ingredients and combinations thereof.

Chelating agents which come into consideration are, in particular, compounds which are able to complex especially polyvalent, in particular divalent and/or trivalent, metal ions in order to avoid an interfering effect of these metal ions on the viscosity of the composition.

Suitable chelating agents can be selected, for example, from the group consisting of ethylene-diaminetetraacetate (EDTA), 1,3-diaminopropane-N,N,N',N'-tetraacetate (DTPA), diethyldithio-carbamate (DEDTC), 2,3-dimercapto-1-propanesulphonic acid (DMPS), ethylenediamine-N,N'-di-β-propionate, 1,2-dimethyl-3-hydroxy-4-pyridinone (DMHP), 1,2-diethyl-3-hydroxy-4-pyridinone (DEHP), ethylmaltol (EM), 4-(6-methoxy-8-quinaldinylaminosulphonyl)benzoate, N-(6-methoxy-8-quinolyl)-*p*-toluenesulphonamide (TSQ), carnosine, deferasirox, *trans*-1,2-cyclohexanediamine-N,N,N',N'-tetraacetate (CyDTA), dihydroxyethylglycine (DHEG), 1,3-diamino-2-hydroxypropane-N,N,N',N'-tetraacetate, (DTPA-OH), ethylenediamine-N,N'-diacetate (EDDA), ethylenediamine-N,N'-dipropionate (EDDP), ethylenediamine-N,N'-bis(methylphosphonate) (EDDPO), N-hydroxyethylenediamine-N,N',N'-triacetate (EDTA-OH), ethylenediaminetetra(methylene)phosphonate (EDTPO), N,N'-bis(2-hydroxybenzyl)ethylenediamine-N,N'-diacetate (HBED), hexamethylene-1,6-diaminetetraacetate (HDTA), hydroxyethyliminodiacetate (HIDA), iminodiacetate (IDA), methyl-EDTA, nitrilotriacetate (NTA), nitrilotripropionate (NTP), nitrilotri(methylene)phosphonate (NTPO), 7,19,30-trioxa-1,4,10,13,16,22,27,33-octaazabicyclo[11.11.11]pentatriacontane (O-bistrene), triethylenetetraaminehexaacetate (TTHA), ethylene glycol-*bis*(2-aminoethyl ether)-N,N,N',N'-tetraacetate (EGTA), dimercaptosuccinate (DMSA), deferoxamine, dimercaprol, citrate, penicillamine, succimer, etidronate, ethylenediamine-di(*o*-hydroxyphenylacetate) (ED-DHA), *trans*-1,2-cyclohexanediaminetetraacetate (CDTA), N-(2-hydroxyethyl)ethylenedinitrilotriacetate (HEDTA), N-(2-hydroxyethyl)iminodiacetate (HEIDA), calprotectin, lactoferrin, ovotransferrin, conalbumin and combinations thereof.

The chelating agent can have a fraction less than 1% by weight, in particular from 0.01% by weight to 0.99% by weight, based on the total weight of the composition.

Suitable preservatives can be selected from the group consisting of methylparaben, ethylparaben, propylparaben, isopropylparaben, butylparaben, benzylparaben, sodium methylparaben, sodium propylparaben, imidazolidinylurea, diazolidinylurea, phenoxyethanol, sodium sulphite, DMDM hydantoin, methylchloroisothiazolinone, methylisothiazolinone, quaternium-15, benzoic acid, sodium benzoate, sorbic acid, potassium sorbate, triclosan, dehydroacetic acid, dehydro-acetate, benzyl alcohol, sodium borate, isobutylparaben, iodopropynyl butylcarbamate, 2-bromo-2-nitropropane-1,3-diol, paraformaldehyde, formalin, salicylic acid, formaldehyde, methyldibromoglutaronitrile, benzalkonium chloride, boric acid, chlorhexidine digluconate, sodium bisulphite, chloroxylenol, hexamidine isethionate, benzethonium chloride, chloroacetamide, methenamine, phenethyl alcohol, 5-bromo-5-nitro-1,3-dioxane, o-phenylphenol, sodium *o-*phenylphenol, sodium hydroxymethylglycinate, grapefruit seed extract, triclocarban, glutaraldehyde, polymethoxybicyclooxazolidine, chlorhexidine dihydrochloride, chlorphenesin, aescin, dichlorobenzyl alcohol, phenylmercury acetate, chlorhexidine acetate, dimethoxane, domiphen bromide, captan, dichlorophen, hinokitiol, *p*-chloro-*m*-cresol, phenoxyisopropanol, thimerosal, chlorobutanol, polyaminopropyl biguanide and combinations thereof.

The preservative can have a fraction less than 1% by weight, in particular from 0.01 % by weight to 0.99% by weight, based on the total weight of the composition.

The detergent builder is preferably one that is able to react with the mucoadhesive component, forming a soap. Owing to the presence of such a detergent builder, particularly advantageously, the homogeneity of the composition may be improved.

Suitable detergent builders can be selected from the group consisting of alkali metal hydroxides, alkali metal carbonates, alkaline earth metal hydroxides, alkaline earth metal carbonates, triethanolamine, triisopropanolamine and combinations thereof.

The detergent builder can have a fraction from 0.1 mol% to 5 mol%, in particular 0.5 mol% to 3 mol%, preferably 1 mol% to 2 mol%, based on the molar amount of the mucoadhesive component.

Suitable active ingredients can be selected from the group consisting of biological active ingredients, medical or pharmaceutical active ingredients and combinations thereof. For example, suitable active ingredients can be selected from the group consisting of antimicrobial, in particular antibiotic, active ingredients, cytostatics, active ingredients promoting wound healing (agents), anti-inflammatory active ingredients, analgesics, disinfectants and combinations thereof.

In an advantageous embodiment, the components necessarily provided of the composition, namely the moisturizing component, the mucoadhesive component, the component obtained from *Aloe vera* and the water, and possibly further optionally provided components, as described, in particular, in the preceding embodiments, are not spatially separated from one another. Complex mixing steps and possibly delayed gelation can be avoided in this manner.

In a further embodiment, the composition can consist of a moisturizing component, a mucoadhesive component, a component obtained from *Aloe vera,* water and possibly other components such as, for example, a chelating agent, a preservative and/or a detergent builder. To this extent, reference is explicitly made to the description thus far.

The composition has preferably at atmospheric pressure in a temperature range from 20 to 37°C a complex viscosity from 1 to 10 Pas, in particular 3 to 8 Pas, preferably 6 to 6.5 Pas. The expression "complex viscosity", for the purposes of the present invention, shall be taken to mean the sum parameter of modulus of elasticity (or storage modulus) and loss modulus. The modulus of elasticity or storage modulus reflects the stored mechanical energy. The loss modulus reflects the dissipatively, i.e. irretrievably, released, viscous fraction of the energy introduced into the composition. The complex viscosity is generally determined by what is termed oscillation measurement. This may be determined, for example, using a rheometer which has a cone-plate arrangement, wherein the composition is generally situated between the cone and the plate. The measurement is carried out under oscillating stress (vibration measurement), wherein the material under test is usually exposed to sinusoidal deformation with low amplitude. The complex viscosity is generally proportional to the phase-delayed oscillating restoring force of the composition, in particular the hydrogel. The low amplitude in this case guarantees measurement in the linear viscoelastic range. The oscillation measurement for determining the complex viscosity of a material is known to those skilled in the art, per se, and so further explanations will be dispensed with at this point.

In a further embodiment, the composition is transparent or translucent. Transparency of the composition is particularly advantageous to the surgeon since, despite the wound area to be treated being covered, the underlying tissue structures can be seen, which is advantageous, in particular, in respect to early recognition of post-operative haemorrhage and/or any dislocation of the composition.

Optionally, the composition can be coloured in a pale yellowish colour.

The composition is preferably sterilized, in particular γ-sterilized.

The composition according to the invention, in a further embodiment, is present in uncrosslinked form, i.e. it is preferably free from physical and/or chemical, in particular covalent, crosslinks.

In addition, the composition can have a resorption time *in vivo* of a maximum of 6 months, in particular 4 to 6 months.

Preferably, the composition, after application thereof, remains stationary for at least 48 hours on an operation site or wound site.

Such an immovability, in particular in combination with the resorption time described in the preceding section, ensures a particularly effective protection against post-surgical adhesions.

The composition can be provided, in particular, for prevention and/or prophylaxis of post-surgical or post-operative tissue adhesions or tissue adherences in the abdominal cavity, in the pelvic cavity, in particular in the region of the internal female genital organs, on the pericardium and/or in the case of damaged nerves.

In a particularly preferred embodiment, the composition according to the invention is the hydrogel commercially available under the name Askina^{®} Gel. This gel is used to date for topological wound treatment. In contrast, its usability for prevention or prophylaxis of post-surgical or post-operative tissue adhesions or tissue growths has not yet been described to date.

A further aspect of the invention relates to a kit which, in addition to the composition according to the invention, comprises at least one further component which is selected from the group consisting of a medical adhesive, a disinfectant, an antibiotic, a cytostatic, an agent promoting wound healing, an implant for tissue regeneration, a haemostyptic, a textile implant, in particular a one-, two-, or three-dimensional textile implant such as, for example, a suture material, a hernia mesh or a plug, an application aid, an open applicator, a laparoscopic applicator, and combinations thereof. With respect to further features and advantages of the kit, in particular with respect to the composition according to the invention, to avoid unnecessary repetitions, reference is made in entirety to the description hereinbefore.

Finally, the present invention also relates to the use of a moisturizing component, a mucoadhesive component, a component obtained from *Aloe vera* and water for producing a composition, preferably in the form of a hydrogel, for use in the prevention and/or prophylaxis of post-surgical or post-operative tissue adhesions or tissue adherences. With respect to further features and advantages of the composition, to avoid unnecessary repetitions, likewise reference is made in entirety to the description hereinbefore.

Finally, the advantages of the composition provided by the invention will be summarized again as follows:
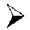 The composition is distinguished in a particularly advantageous manner by pronounced biocompatibility.
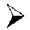 A further advantage is that the composition, after application thereof onto an operation site or wound site, exhibits sufficiently long immovability in such a manner that unwanted dislocations and as a consequence tissue adhesions cannot occur.
   The immovability of the composition which is favourable in respect of avoiding tissue adhesions is achieved in particular by the presence of the mucoadhesive component within the composition.
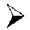 A further advantage is that the composition is distinguished by a sufficiently long, adhesion-preventing resorption time *in vivo.*
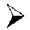 In addition, the composition is distinguished particularly advantageously by a certain thermostability. For instance, the composition can be thermally stable within a comparatively broad temperature range, in particular within a temperature range of -20°C to 60°C, in particular can have a constant or substantially constant complex viscosity. This in turn improves the handling of the composition, for example from storage, transport and/or application aspects, and thereby increases the safety in use because of the absence of dislocation of the composition. Particularly advantageously, the composition possesses a constant or substantially constant complex viscosity in a temperature range from 20 to 37°C.
   A constant or substantially constant viscosity in addition has the advantage that it (in addition to the mucoadhesive component) improves the immovability of the composition on an operation site or wound site.
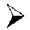 A further advantage relates to the γ-sterilizability of the composition, without thickening or curing of the composition occurring owing to crosslinking. This in turn permits a very inexpensive production of a sterile composition.

Further features and advantages of the invention result from the subsequent description of preferred embodiments in the form of examples and also in combination with the features of the dependent claims. In these embodiments, individual features of the invention can be realized alone or in combination with other features. The preferred embodiments described are only to be understood as descriptive disclosure, but in no way as a limiting disclosure.

### Example Section

### Example 1: Determination of Complex Viscosity

The complex viscosity of the hydrogel commercially available under the name Askina^{®} Gel (from Aesculap AG) was determined using a Bohlin rheometer of the Gemini 150 type having a cone-plate system.

Single determination at constant frequency of three hydrogel samples stored at differing temperatures was performed.

The plate had a diameter of 40 mm and the gap angle was 4°. The gap distance was 150 µm.

The following samples were used:
Sample 1: Askina^{®} Gel, one week at room temperature (atmospheric pressure)
Sample 2: Askina^{®} Gel, one week at -20°C (atmospheric pressure)
Sample 3: Askina^{®} Gel, one week at +37°C (atmospheric pressure)

The samples were each stored in tubes having a sealed closure cap (contents 15 g).

The following parameters were used as the basis for the viscosity determination:
Temperature: isothermal 22°C (samples 1 and 2) or 37°C (sample 3)
Temperature equilibration time: 900 s
Frequency: 10 Hz
Deformation: 0.003 s
Stationary shear rate: 0 1/s
Integration time: 4 s
Integration periods: 40
Points: 2048
Number of measurements: 30
Delay time: 2 s
Time per point: 10 s
Continuous oscillation: on
Total measurement time: 300 s

The values listed in Table 1 below for the complex viscosity of the hydrogels were obtained:

**Table 1: Determination of complex viscosity**

| Hydrogel | Complex Viscosity [Pas] (standard deviation SD) |
|---|---|
| Sample 1 | 6.30 (SD = 0.20) |
| Sample 2 | 6.32 (SD = 0.16) |
| Sample 3 | 6.45 (SD = 0.17) |

It can be seen from the table that the viscosities of the hydrogels were substantially constant independently of the storage conditions.

### Example 2: Adhesion prophylaxis - abdominal wall defect model, functionality and biocompatibility in intraabdominal use on rabbits

Within the framework of an animal study, the functionality of Askina^{®} Gel was studied with respect to prevention of the occurrence of intraabdominal adhesions and also local compatibility thereof.

The study was carried out on 17 rabbits (SPF albino rabbits of the race Crl:CHBB(HM) Russian rabbits; Charles River Deutschland GmbH), wherein all animals reached the specified end of study of 21 days *post operationem.*

Whereas 5 animals were treated with Askina^{®} Gel, 12 animals formed a control group, which were subjected to the same procedure, but without Askina^{®} Gel treatment.

In the case of the abovementioned five animals, after preparation of an abdominal wall defect and also a blunt traumatization (Burns JW, Skinner K, Colt MJ, Burges L, Rose R, Diamond MP, A hyaluronate based gel for the prevention of postsurgical adhesions: evaluation in two animal species. Fertility and Sterility: 1996; 66: 814-821) of the adjacent caecum was performed (by means of a sterile gauze compress), the traumatized abdominal wall area (defect area: approximately 2.5 x 2.5 cm) was treated with Askina^{®} Gel (gel area: approximately 3.0 x 3.0 cm).

The abdominal wall defect area, the liver, the kidneys and the spleen were removed 21 days *post operationem* for a histological examination.

The results of the findings obtained here are reproduced in the table hereinafter.

**Table 2: Occurrence of adhesions with the use of Askina^{®} Gel**

| Animal | Adhesions in the region of the defect | Adhesions in the adjacent edge area of the defect | Further intraabdominal adhesions |
|---|---|---|---|
| 1 | 100% free | Yes (ventral edge area) | Caecum with muscle suture and abdominal wall |
| 2 | 100% free | Yes (ventral edge area) | Caecum with muscle suture and abdominal wall, caecum with omentum majus |
| 3 | 100% free | No | Caecum with muscle suture, caecum with omentum majus and colon |
| 4 | 85% free | Yes (ventral and cranioventral edge area) | Caecum with muscle suture and abdominal wall, caecum with omentum majus |
| 5 | 100% free | No | Caecum with omentum majus |

In four of five animals, the abdominal wall defect was entirely free from adhesions. In one animal, the abdominal wall defect area was anyway 85% free from adhesions. In all animals, the test object was in phagocytosis or resorption. The course of healing of the abdominal wall defect was able to be rated good after 21 days and on account of the local to moderate tissue reaction on the omentum, abdominal wall defect and caecum, there was good biocompatibility. The follicular hyperplasia of the spleen observed in all animals represented a physiological immunological reaction to the lesions performed and to the test object introduced, and were not considered to be critical.

The results obtained therefore impressively confirm the adhesion prophylaxis potential of Askina^{®} Gel. This is supported in addition by the observation that adhesions between the caecum and the muscle suture and also adjoining abdominal wall were present, which may be explained by the fact that the Askina^{®} Gel was applied solely to the abdominal wall defect area (3.0 x 3.0 cm).

## Claims

1. Composition, preferably in the form of a hydrogel, for use in the prevention and/or prophylaxis of post-surgical tissue adhesions, comprising a moisturizing component, a mucoadhesive component, a component obtained from *Aloe vera* and water.

2. Composition according to Claim 1, **characterized in that** the moisturizing component is a polyol, in particular di- or triol, preferably glycerol.

3. Composition according to Claim 1 or 2, **characterized in that** the moisturizing component has a fraction of 5% by weight to 20% by weight, in particular 5% by weight to 15% by weight, preferably 8% by weight to 12% by weight, based on the total weight of the composition.

4. Composition according to any one of the preceding claims, **characterized in that** the mucoadhesive component is a polycarboxylic acid, in particular polyacrylic acid.

5. Composition according to any one of the preceding claims, **characterized in that** the mucoadhesive component has a fraction of 0.1% by weight to 5% by weight, in particular 0.5% by weight to 3% by weight, preferably 1% by weight to 2% by weight, based on the total weight of the composition.

6. Composition according to any one of the preceding claims, **characterized in that** the component obtained from *Aloe vera* is selected from the group consisting of *Aloe vera* gel, *Aloe vera* extract, *Aloe vera* powder and combinations thereof.

7. Composition according to any one of the preceding claims, **characterized in that** the component obtained from *Aloe vera* has a fraction of 0.1 % by weight to 5% by weight, in particular 0.5% by weight to 3% by weight, preferably 1% by weight to 2% by weight, based on the total weight of the composition.

8. Composition according to any one of the preceding claims, **characterized in that** the water has a fraction of 65% by weight to 90% by weight, in particular 80% by weight to 90% by weight, preferably 80% by weight to 85% by weight, based on the total weight of the composition.

9. Composition according to any one of the preceding claims, **characterized in that** the composition comprises a further component selected from the group consisting of chelate formers, preservatives, detergent builders and combinations thereof.

10. Composition according to any one of the preceding claims, **characterized in that** the composition is transparent.

11. Composition according to any one of the preceding claims, **characterized in that** the composition is sterilized, preferably γ-sterilized.

12. Composition according to any one of the preceding claims, **characterized in that** the composition is in non-crosslinked form, in particular is free from physical and/or chemical, in particular covalent, crosslinks.

13. Kit for use in the prevention and/or prophylaxis of post-surgical tissue adhesions, comprising, in addition to a composition according to any one of the preceding claims, at least one further component which is selected from the group consisting of a medical adhesive, a disinfectant, an antibiotic, a cytostatic, an agent promoting wound healing, an implant for tissue regeneration, a haemostyptic, a one-, two-, or three-dimensional textile implant such as, for example, a suture material, a hernia mesh or a plug, an application aid, an open applicator, an applicator for laparoscopic purposes, and combinations thereof.
